# EUROPEAN PATENT SPECIFICATION

(11) **EP 1 081 140 B1**
(45) Date of publication and mention of the grant of the patent: **01.06.2005**
(21) Application number: 00118363.1
(22) Date of filing: 24.08.2000
(51) Int. Cl.: C07D 239/62, C07F 7/10, C09K 15/30, C07D 239/60, C08G 77/388

(54) **Novel Pyrimidine-2,4,6-trione compounds**
Pyrimidine-2,4,6-trione Verbindungen
Composés de pyrimidine-2,4,6-trione

(30) Priority: 01.09.1999 EP 99117187
(43) Date of publication of application: 07.03.2001
(73) Proprietor: DSM IP Assets B.V., 6411 TE Heerlen (NL)
(72) Inventor: Huber, Ulrich, 8703 Erlenbach (CH)
(74) Representative: Schwander, Kuno Josef, Dr.

(56) References cited:
- WO-A-98/14423
- US-A- 5 561 179
- CHEMICAL ABSTRACTS, vol. 130, no. 24, 1999 Columbus, Ohio, US; abstract no. 325830s, page 653; XP002160718 & JP 11 116556 A (CHEMIPRO KASEI) 27 April 1999 (1999-04-27)
- CHEMICAL ABSTRACTS, vol. 130, no. 19, 1999 Columbus, Ohio, US; abstract no. 253159e, page 820; XP002160719 & JP 01 180130 A (CHEMIPRO KASEI) 18 July 1989 (1989-07-18)
- CHEMICAL ABSTRACTS, vol. 131, no. 17, 1999 Columbus, Ohio, US; abstract no. 228730x, page 715; XP002160720 & JP 11 263778 A (CHEMIPRO KASEI) 28 September 1999 (1999-09-28)

## Description

The present invention relates to novel pyrimidine-2,4,6-trione compounds which are effective in absorbing ultra violet radiation and to light screening compositions comprising said pyrimidine-2,4,6-trione compounds.

US 5,561,179 discloses piperidine compounds containing silane groups as stabilizers for organic materials as well as their use as light, heat and oxidation stabilizers for organic materials.

JP 11-116556 discloses aminomethylenepyrimidine derivatives having an ultraviolet light absorption capacity in the UV-A region, as well as ultraviolet light absorbing agents, cosmetic materials and organic polymer compositions containing same.

JP 11-80130 discloses aminomethylenepyrimidine derivatives having an ultraviolet light absorption capacity, little volatility/fugacity and high stability, as well as ultraviolet light absorbing agents and weather-resistant organic polymer compositions containing same.

Light screening compositions comprising pyrimidine-2,4,6-trione compounds are described in the International Publication WO 98/14423. This publication refers inter alia to compounds of the general formula 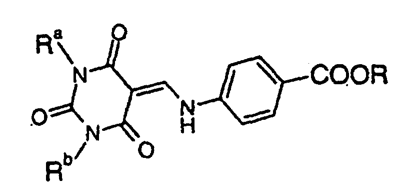 wherein
- R: is alkyl, cyclic alkyl, aralkyl or aryl;
- R^{a} and R^{b}: each independently are hydrogen, alkyl, cyclic alkyl, aralkyl and aryl.

The quoted International Publication teaches that these claimed compounds have an excellent ultraviolet absorbing ability. However, these compounds are sensitive to hydrolytic decomposition.

It has now been found that compounds of the above formula but wherein the group -COOR is replaced by an ether group -OR or an ester group -OCOR exhibit superior chemical stability.

Thus, the invention relates to compounds of the general formula I 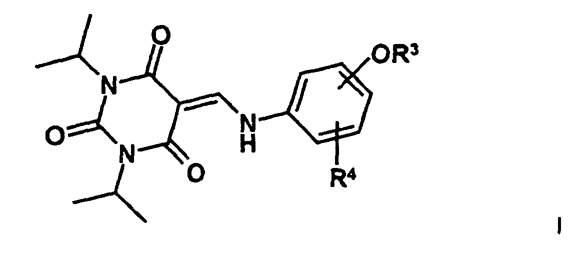 wherein
- R³: is hydrogen, C₁-C₁₈ alkyl or C₂-C₁₈ alkyl in which at least one methylene group is replaced by oxygen, or is C₁-C₁₈ alkyl carbonyl, C₃-C₁₈ alkenyl, C₃-C₁₈ alkynyl or a group YS;
- R⁴: is hydrogen, C₁-C₈ alkyl or a group OR³;
- Y: is a linker group which is a C₃-C₁₂ divalent alkylene or alkenylene chain each of which may optionally be interrupted by one or several oxygen atoms; and
- S: is a silane-, an oligosiloxane- or a polysiloxane moiety, wherein the silane moiety is a group -SiR⁵R⁶R⁷, wherein R⁵, R⁶ and R⁷ each independently are C₁-C₆ alkyl or phenyl,
the oligosiloxane moiety is a group of the general formula -SiR⁸ₘ(OSiR^{8'}₃)ₙ with m = 0, 1 or 2; n = 1, 2 or 3 and m + n = 3, wherein R⁸ and R^{8'} each independently are C₁-C₆ alkyl or phenyl, or a group of the general formulae IIa or IIb 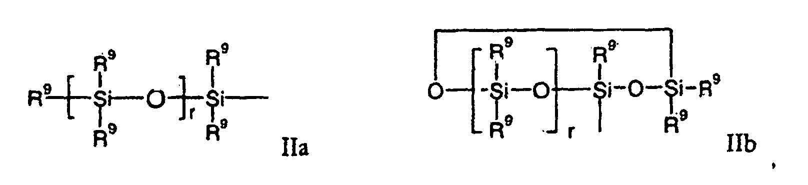
wherein R⁹ is C₁-C₆ alkyl or phenyl and r is an integer from 1 to 9,
and the polysiloxane moiety is a group of the general formulae IIIa or IIIb, 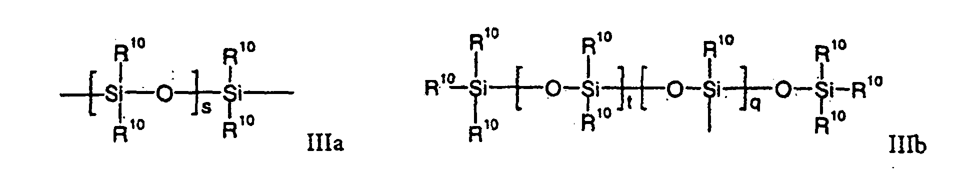 wherein
- R¹⁰: is C₁-C₆ alkyl or phenyl;
- s: is an integer from 4 to 250;
- t: is an integer from 5 to 250;
- q: is an integer from 1 to 30.

The invention further relates to light screening compositions comprising a compound of formula I, and to the use of a compound of formula I as an agent for absorbing ultraviolet light.
In the compounds of formula I, the residue OR³ is preferably in the para position. R³ preferably is C₂-C₈ alkyl or a group YS, more preferably 2-ethyl hexyl or a group YS.

The residue R⁴ preferably is hydrogen or hydroxy.

The term "C₁-C₁₈ alkyl" refers in the present context to straight chain or branched saturated hydrocarbon residues with 1 to 18 carbon atoms such as methyl, ethyl, propyl, isopropyl, thexyl, (1,1,2 dimethylpropyl), n-butyl, sec. butyl, tert. butyl, pentyl, neopentyl, hexyl, 2-ethyl-hexyl, octyl and the like.

The term "C₂-C₁₈ alkyl in which at least one methylene group is replaced by oxygen" refers in the present context to straight chain or branched saturated hydrocarbon residues with up to 17 carbon atoms which are bound via a carbon atom and have at least one group such as -(CH₂-O)-, -(CH₂-CH₂-O)-, -(CH₂-CH₂-CH₂-O)-, -(CH₂-CH₂CH₂-CH₂-O)-and the like.

In the term "C₁-C₁₈ alkyl carbonyl" the alkyl residue is as defined above.

The term "C₃-C₁₈ alkenyl" refers in this context to straight chain or branched unsaturated hydrocarbon residues with 3 to 18 carbon atoms containing at least one double bond such as propen-2-yl, propen-3-yl, buten-3-yl, buten-4-yl, penten-4-yl, penten-5-yl and the like.

The term "C₃-C₁₈ alkynyl" refers in this context to straight chain or branched unsaturated hydrocarbon residues with 3 to 18 carbon atoms containing at least one triple bond. A preferred alkynyl residue is propargyl.

The term "linker group" refers in this context to a C₃-C₁₂ divalent alkylene or alkenylene chain which links the UV absorbing chromophoric residue to the silane, oligosiloxane or polysiloxane moiety.

The term "C₃-C₁₂ divalent alkylene chain" refers in this context to straight chain or branched saturated hydrocarbon residues such as 3-propylene, 2-propylene, 2-methyl-3-propylene, 3-butylene, 4-butylene, 4-pentylene, 5-pentylene, 6-hexylene and the like.

The term "C₃-C₁₂ divalent alkenylene chain" refers in this context to unsaturated hydrocarbon residues containing at least one double bond such as 2-propen-2-ylene, 2-propen-3-ylene, 3-buten-3-ylene 3-buten-4-ylene, 4-penten-4-ylene, 4-penten-5-ylene, (3-methyl)-penta-2,4-dien-4 or 5-ylene, 11-dodecen-11-ylene and the like.

The divalent alkylene or alkenylene chains may be interrupted by one or several oxygen atoms. Examples for by oxygen interrupted divalent alkylene or alkenylene chains are e.g. 2-ethyloxy-eth-2-ylene, 4-butyloxy-eth-2-ylene or 3,6-dioxa-8-octylen.

Preferred divalent alkylene or alkenylene chains are 3-propylene, 4-butylene, 2-propen-2-ylene, 2-propen-3-ylene or 3-buten-4-ylene. Especially preferred is 2-propen-2-ylene or 2-propen-3-ylene.

The term "silanemoiety" refers in this context to a group -SiR⁵R⁶R⁷, wherein R⁵, R⁶ and R⁷ each independently are C₁-C₆ alkyl or phenyl. Preferred silane moieties are e.g. trimethylsilane, triethylsilane, tripropylsilane, triisopropylsilane, dimethyl tert.butylsilane, dimethyl thexylsilane, triphenylsilane, dimethylphenylsilane and the like.

The term "oligosiloxane moiety" refers in this context to groups of the general formula -SiR⁸ₘ(OSiR^{8'}₃)ₙ with m = 0, 1 or 2; n = 1, 2 or 3 and m+n = 3, wherein R⁸ and R^{8'} each independently are C₁-C₆ alkyl or phenyl. Preferably R⁸ and R^{8'} have the same meaning. Preferred is the group -SiMe(OSiMe₃)₂, wherein Me signified methyl.

The term "oligosioxane" further refers to groups of the general formula IIa or IIb 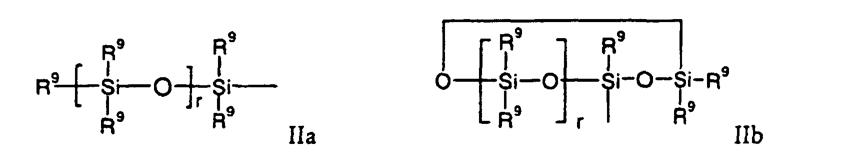 wherein R⁹ is C₁-C₆ alkyl or phenyl and r is an integer from 1 to 9, preferably 1 to 3. The residue R⁹ is preferably C₁-C₄ alkyl, more preferably methyl.

The term "polysiloxane moiety" refers in this context to groups of the general formulae IIIa or IIIb, 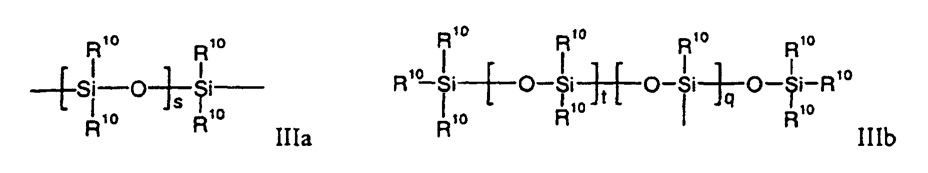 wherein
- R¹⁰: is C₁-C₆ alkyl or phenyl;
- s: is an integer from 4 to 250;
- t: is an integer from 5 to 250;
- q: is an integer from 1 to 30.

Preferably, "s" is an integer from 4 to 150.

Preferably, "q" is an integer from 2 to 10, more preferably has statistical mean value of about 4.

Preferably, "t" is an integer from 5 to 150, more preferably has statistical mean value of about 60.

The residue R¹⁰ is preferably C₁-C₄ alkyl, more preferably methyl.

The compounds of the general formula I can be prepared by methods known per se.

For compounds of the general formula I, wherein R³ is C₁-C₁₈ alkyl, C₂-C₁₈ alkyl in which at least one methylene group is replaced by oxygen, C₃-C₁₈ alkenyl or C₃-C₁₈ alkynyl and R⁴ is hydrogen or C₁-C₈ alkyl the process starts by alkylation of a nitrophenol of the formula IVa 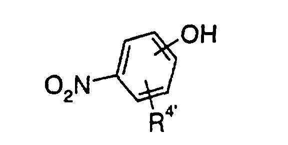 wherein
R^{4'} is hydrogen or C₁-C₈ alkyl;
with an alkyl halide to provide a nitro phenolic ether IVb.

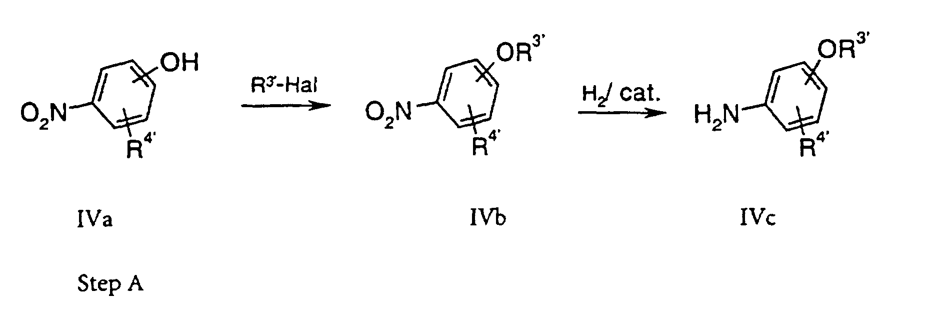

The alkyl halide has the formula R^{3'}Hal, wherein R^{3'} is C₁-C₁₈ alkyl, C₂-C₁₈ alkyl in which at least one methylene group is replaced by oxygen, C₃-C₁₈ alkenyl or C₃-C₁₈ alkynyl; and Hal is chloro, bromo or iodo, preferably, chloro or bromo.

The starting nitrophenol is commercially available or may be readily prepared by known methods.

The reaction is run in a suitable reaction solvent. The choice of the solvent is not critical. Suitable solvents include toluene, pyridine or polar aprotic solvents such as e.g. 1-methyl-2-pyrrolidone, dimethylformamide, dimethylsulfoxide, dioxane, tetrahydrofuran, acetonitrile and the like. The reaction mixture includes an acid acceptor, such as a mild base, for example, sodium or potassium carbonate, which can absorb the acid-by-product of the alkylation. The reactants usually are present in about equal molar amounts and the reaction is run at elevated temperature, e.g. at about 80° C-150°C, preferably at about 100° C.

The nitro phenolic ether IVb obtained is then reduced either by conventional methods using reduction with tin or zinc and hydrochloric acid or by catalytic hydrogenation to afford the corresponding amino group to provide p-amino phenolic ether of the formula IVc. The catalytic hydrogenation is employed, using conventional catalysts such as Raney-nickel, palladium or platinum. The catalytic hydrogenation is carried out at temperatures in the range of 0°C to about 100° C, preferably at about 20°C to about 60°C, more preferably at about 40° C and under atmospheric pressure to about 100 bar, preferably at about 20 bar to about 60 bar, more preferably at about 40 bar.

Nitro phenolic ether compounds wherein R^{3'} is C₃-C₁₈ alkenyl or C₃-C₁₈ alkynyl are preferably reduced using tin and hydrochloric acid.

Compounds of the general formula I, wherein R³ is C₁-C₁₈ alkyl, C₂-C₁₈ alkyl in which at least one methylene group is replaced by oxygen, C₃-C₁₈ alkenyl or C₃-C₁₈ alkynyl and R⁴ is a group OR^{3'} can be prepared accordingly. 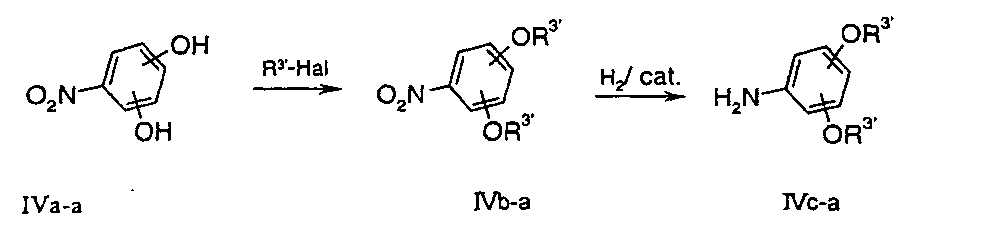 The group R³ is as defined above.

Compounds of the general formula I, wherein R³ is C₁-C₁₈ alkyl, C₂-C₁₈ alkyl in which at least one methylene group is replaced by oxygen, C₃-C₁₈ alkenyl or C₃-C₁₈ alkynyl and R⁴ is hydroxy (OR³ with R³=H) can be prepared using a conventional protective group for the hydroxy group such as e.g. ether-type protective groups or acetal-type protective groups.

Compounds of the general formula I, wherein R³ is hydrogen and R⁴ is hydrogen or C₁-C₈ alkyl can be prepared by reducing the corresponding nitro phenol IVa-b using conventional methods.

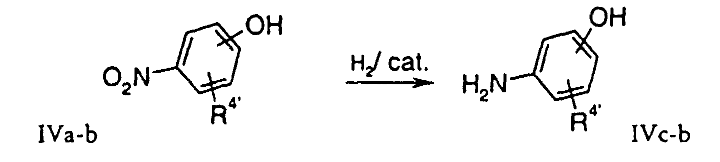

R⁴ is as defined above.

The pyrimidine-2,4,6-trione residue of the general formula V 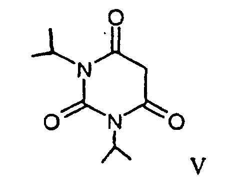 can be prepared by adding malonic acid in an equimolar quantity to a carbodiimide of the formula >-N=C=N-<. The reaction is run in a suitable reaction solvent. The choice of the solvent is not critical. Preferred are polar aprotic solvents as listed above.

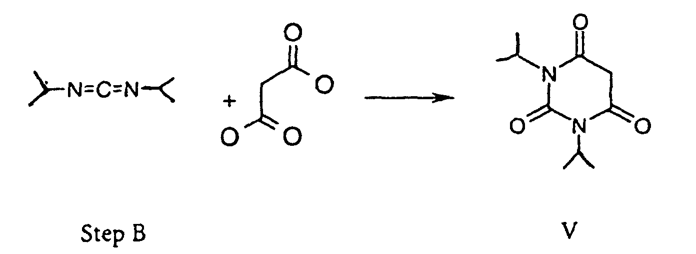

Other methods to prepare the pyrimidine-2,4,6-trione residue of the general formula V, starting from e.g. malonic ester and dialkyl urea are described in the International Publication WO 95/00112.

Finally residue V is linked with residue Vlby condensation in the presence of trialkyl orthoformate, preferably triethyl orthoformate [CH(OC₂H₅)₃] to form compound VII 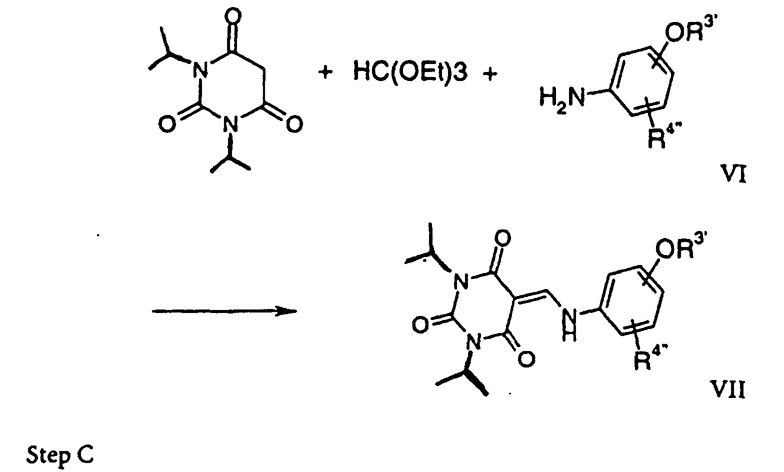 wherein R^{3'} is as defined above, and R^{4"} is hydrogen, C₁-C₈ alkyl or OR^{3'}.

The condensation is carried out in a solvent at reflux temperatures for several hours. The choice of the solvent is not critical. Preferred are polar aprotic solvents as listed above.

For preparing a compound of the general formula I it is also possible to first prepare an intermediate of the general formula VIIa 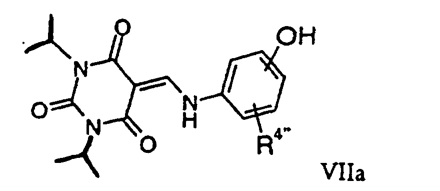 wherein R^{4"} is as defined above by reacting a amino phenol with a pyrimidine 2,4,6-trione compound in the presence of trialkyl orthoformate, preferably triethyl orthoformate [CH(OC₂H₅)₃] and then alkylating the resulting intermediate of the formula VIIa.

Compounds of the general formula I, wherein R³ is C₁-C₁₈ alkyl carbonyl and R⁴ is hydrogen or C₁-C₈ alkyl can be prepared by acylating the above compound of the general formula VIIa using known esterification reactions.

Compounds of the general formula I containing a silane , an oligosiloxane or a polysiloxane moiety are prepared by hydrosilation. The following reaction scheme shows an example to prepare a compound of the general formula I wherein R³ is a group YS wherein S is a an oligosiloxane of the formula -SiR⁸ₘ(OSiR^{8'}₃)ₙ with m = 0, 1 or 2; n = 1, 2 or 3 and m+n = 3, wherein R⁸ and R^{8'} each independently are C₁-C₆ alkyl or phenyl and R^{4'} is as defined above, namely hydrogen or C₁-C₈ alkyl.

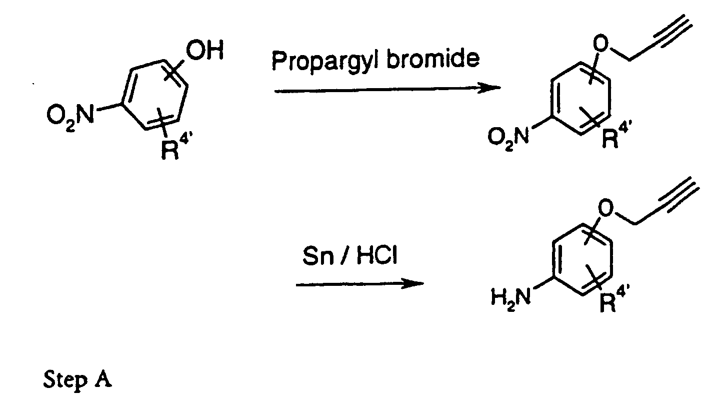

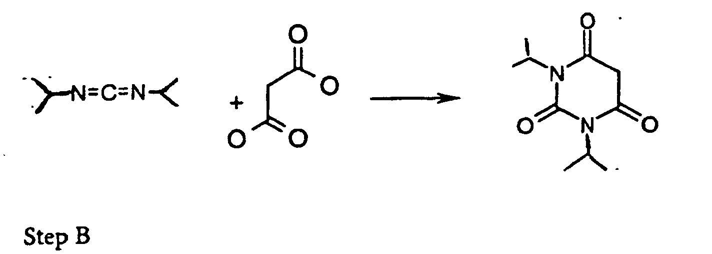

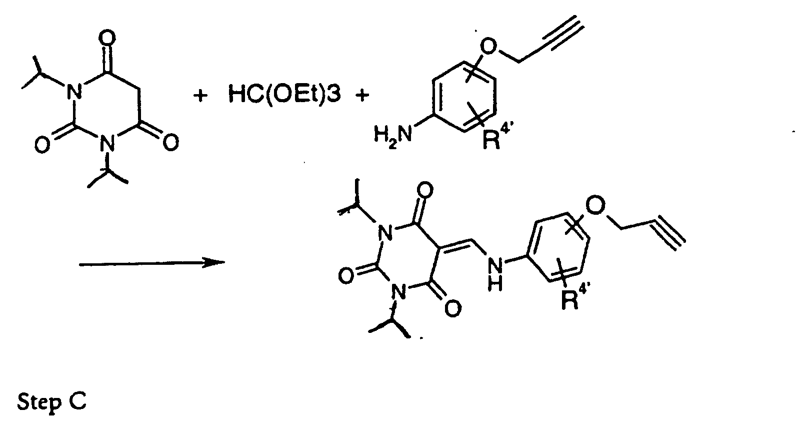

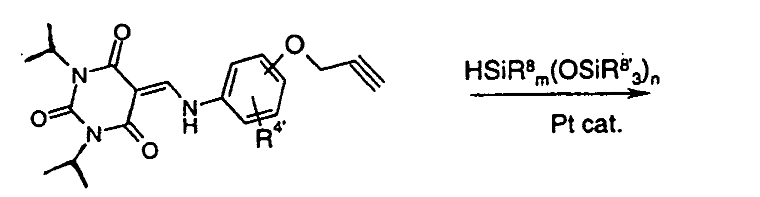

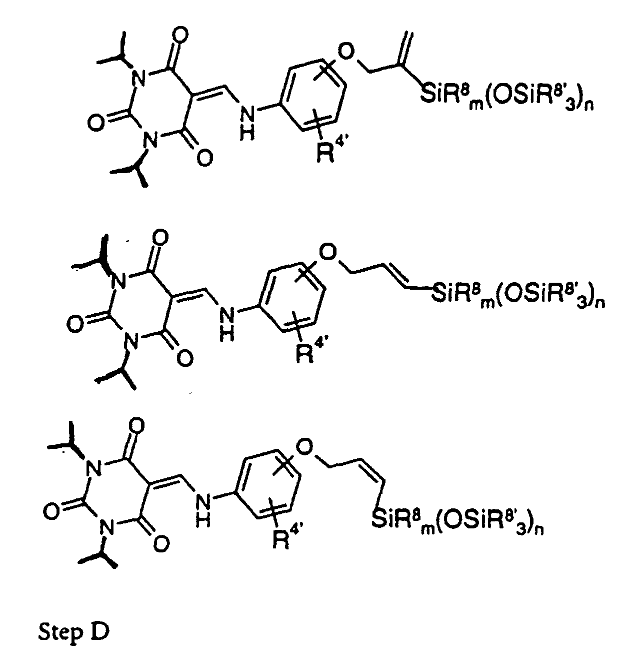

The hydrosilation reaction between the SiH group and the alkynyl group (step D) is carried out in the presence of a transition metal catalyst e.g. platinum on charcoal or a platinum complex catalyst such as e.g. divinyl-tetramethyl disiloxane platinum complex. The reaction is run in a suitable reaction solvent such as e.g. in toluene. The reactants usually are present in about equal molar amounts and the reaction is run at a somewhat elevated temperature, e.g. at about 60°C-150°C, preferably at about 40°C-100°C, more preferably at about 80° C.

Compounds of the general formula I containing an oligosiloxane moiety of the general formulae Ila or Ilb or a polysiloxane moiety of the general formulae IIIa or Illb are prepared accordingly. Reaction partner is the corresponding SiH containing oligosiloxane or polysiloxane moiety.

The compounds of the general formula I have adsorption maxima in the range of 320 nm to 400 nm, the so called UV-A radiation.

The preparation of novel light screening agents, especially of preparations for skin protection and, respectively, sunscreen preparations for everyday cosmetics, comprises incorporating a compound of formula I in a cosmetic base which is conventional for light screening agents. Where convenient, other conventional UV-A, and respectively, UV-B filters can also be combined during this incorporation. Said combinations of UV filters can show a synergistic effect. The preparation of said light screening agents is well known to the skilled artisan in this field. The amount of compounds of the general formula I and other known UV-filters is not critical. Suitable amounts are about 0.5 to about 12%.

Suitable UV B filters, i.e. substances having absorption maxima between about 290 and 320 nm, are for example the following organic compounds which belong to the widest classes of substance:
--- Acrylates such as 2-ethylhexyl 2-cyano-3,3-diphenylacrylate (octocrylene, PARSOL 340), ethyl 2-cyano-3,3-diphenylacrylate and the like;
--- Camphor derivatives such as 4-methyl benzylidene camphor (PARSOL 5000), 3-benzylidene camphor, camphor benzalkonium methosulfate, polyacrylamidomethyl benzylidene camphor, sulfo benzylidene camphor, sulphomethyl benzylidene camphor, therephthalidene dicamphor sulfonic acid and the like;
--- Cinnamate derivatives such as octyl methoxycinnamate (PARSOL MCX), ethoxyethyl methoxycinnamate, diethanolamine methoxycinnamate (PARSOL Hydro), isoamyl methoxycinnamate and the like as well as cinnamic acid derivatives bond to siloxanes;
--- Organosiloxane compounds containing benzmalonate groups as described in the European Patent Publications EP 0358584 B1, EP 0538431 B1 and EP 0709080 A1;
--- Pigments such as microparticulated TiO₂, and the like. The term "microparticulated" refers to a particle size from about 5 nm to about 200 nm, particularly from about 15 nm to about 100 nm. The TiO₂ particles may also be coated by metal oxides such as e.g. aluminum or zirconium oxides or by organic coatings such as e.g. polyols, methicone, aluminum stearate, alkyl silane. Such coatings are well known in the art.
--- Imidazole derivatives such as e.g. 2-phenyl benzimidazole sulfonic acid and its salts (PARSOL HS). Salts of 2-phenyl benzimidazole sulfonic acid are e.g. alkali salts such as sodium- or potassium salts, ammonium salts, morpholine salts, salts of primary, sec. and tert. amines like monoethanolamine salts, diethanolamine salts and the like.
--- Salicylate derivatives such as isopropylbenzyl salicylate, benzyl salicylate, butyl salicylate, octyl salicylate (NEO HELIOPAN OS), isooctyl salicylate or homomenthyl salicylate (homosalate, HELIOPAN) and the like;
--- Triazone derivatives such as octyl triazone (UVINUL T-150), dioctyl butamido triazone (UVASORB HEB) and the like.

Suitable UV A filters i.e. substances having absorption maxima between about 320 and 400 nm, refers to the following UV-A screening agents.
---- Dibenzoylmethane derivatives such as 4-tert. butyl-4'-methoxydibenzoylmethane (PARSOL 1789), dimethoxydibenzoylmethane, isopropyldibenzoylmethane and the like;
---- Benzotriazole derivatives such as 2,2'-methylene-bis-(6-(2H-benzotriazole-2-yl)-4-(1,1,3,3,-tetramethylbutyl)-phenol (TINOSORB M) and the like;
---- Pigments such as microparticulated ZnO and the like. The term "microparticulated" refers to a particle size from about 5 nm to about 200 nm, particularly from about 15 nm to about 100 nm. The ZnO particles may also be coated by metal oxides such as e.g. aluminum or zirconium oxides or by organic coatings such as e.g. polyols, methicone, aluminum stearate, alkyl silane. Such coatings are well known in the art.

As dibenzoylmethane derivatives are photolabile it is necessary to photostabilize these UV-A screening agents. Thus, the term "conventional UV-A screening agent" also refers to dibenzoylmethane derivatives such as e.g. PARSOL 1789 stabilized by the following stabilizing agents:
---- 3,3-Diphenylacrylate derivatives as described in the European Patent Publications EP 0 514 491 B1 and EP 0 780 119 A1;
---- Benzylidene camphor derivatives as described in the US Patent No. 5,605,680;
---- Organosiloxanes containing benzmalonate groups as described in the European Patent Publications EP 0358584 B1, EP 0538431 B1 and EP 0709080 A1.

As cosmetic bases conventional for light screening compositions in the scope of the present invention there can be used any conventional preparation which corresponds to the cosmetic requirements, e.g. creams, lotions, emulsions, salves, gels, solutions, sprays, sticks and milks; see also, Sunscreens, Development, Evaluation and Regulatory Aspects, ed. N.Y. Lowe, N.A. Shaath, Marcel Dekker, Inc. New York and Basel, 1990.

Having regard to their good lipophility, the compounds of the general formula I can be incorporated well into oil and fat containing cosmetic preparations.

The following examples illustrate the invention in more detail, but do not limit its scope in any manner.

### Example 1

### 5-{[4-(2-ethyl-hexyloxy)-phenylamine]-methylene}-1,3-diisopropyl-pyrimidine-2,4,6-trione.

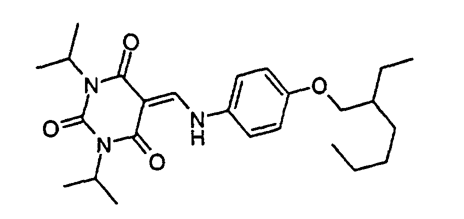

### a) 4-Nitrophenyl-(2-ethyl-hexyl)-ether

In a 300 ml three necked reaction flask equipped with a reflux condensor, a thermometer and a magnetic stirrer 27.7g of p-nitrophenol and 52.7g of anhydrous sodium carbonate were suspended in 180 ml 1-methyl-2-pyrolidone under nitrogen atmosphere. 48 ml of 2-ethyl-hexylbromide were added by means of a dropping funnel. The reaction mixture was heated to 100° C for 180 min. Then the mixture was pored into water and extracted four times with ethyl acetate. The combined organic phases were washed with 1n NaOH and with NaCl solution, dried with Na₂SO₄ followed by concentration at a rotary evaporator. The product was freed of the residual 2-ethyl-hexylbromide by heating to 60° C at a high vacuum of 0.5 mbar. Yield 32.7g (69% of the theory) of a clear yellow liquid. MS: 192(M+), 163, 113, 83, 71, 57(100%).

### b) 4-(2-Ethyl-hexyloxy)-phenylamine

A 600 ml hydrogenation autoclave was charged with 36 g (0.143 mol) of the above p-nitro phenolic ether and with 5 g of wet Raney Ni catalyst in 50 ml methanol. The reduction was carried out for 18 hours at 40° C and a hydrogen pressure of 40 bar. The mixture was filtered and concentrated, followed by a flash chromatography in hexane/ethyl acetate = 9:1 and "Kugelrohr" distillation (200° C /0.8 mbar). Yield 8.8g of a brownish liquid.

### c) 1,3-diisopropyl-pyrimidine-2,4,6-trione.

13.4g of malonic acid was suspended in 130 ml of tetrahydrofuran in a 500ml reaction flask and cooled to 0° C. A solution of 32.6g of N,N'-diisopropyl-carbodiimide in 130 ml of tetrahydrofuran was slowly added within 45 min. at 0°C to 5°C followed by stirring for 2 hours at this temperature. The reaction mixture was concentrated at a rotary evaporator and recrystallized in cyclohexane and then in hexane. Yield 20.6g (75% of the theory) of slightly yellowish crystals, m.p. 145 - 148°C.

### d) 5-{[4-(2-ethyl-hexyloxy)-phenylamine]-methylene}-1,3-diisopropyl-pyrimidine-2,4,6-trione.

A 100 ml three necked reaction flask equipped with a thermometer a reflux condensor and a magnetic stirrer under nitrogen atmosphere was charged with 1.1g (5 mmol) of 4-(2-ethyl-hexyloxy)-phenylamine (Example 1b), 1.06g (5 mmol) of 1,3-diisopropyl-pyrimidine-2,4,6-trione (Example 1c) and 11.1 g (75 mmol) of triethyl orthoformate in 10 ml of tetrahydrofuran. This mixture was refluxed for 18 hours followed by washing twice with water, dried with Na₂SO₄ and concentrated at a rotary evaporator. The raw product was recrystallized in ethanol to yield 0.84 g of yellowish crystals. M.p. 74 - 76° C, UV 355 nm (32'122). This product was irradiated in high dilution with a Hg-lamp 150 W from Hereus and has been shown to be photostable.

### e) Measurement of solubility in Cétiol LC (cocoyl caprylate caprate) and Crodamol DA (diisopropyl adipate).

Oversaturated solutions of 5-{[4-(2-ethyl-hexyloxy)-phenylamine]-methylene}-1,3-diisopropyl-pyrimidine-2,4,6-trione (see above) in the above solvents were prepared and treated in the ultrasonic bath for five minutes. After standing over night at 25° C the solution was filtered through a microfilter (Millipore, pore size 0.5 µm), followed by UV measurement in CH₂Cl₂. The extinctions were compared with the extinctions of the pure compound. The solubilities were found to be 15.5% in Cétiol LC and 26.9% in Crodamol DA.

### Example 2

### 5-{(4-propargyloxy-phenylamine)-methylene}-1,3-diisopropyl-pyrimidine-2,4,6-trione.

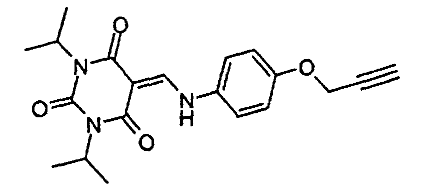

### a) 4-Nitrophenyl-propargylic-ether

In a 200 ml three necked reaction flask equipped with a reflux condensor, a thermometer and a magnetic stirrer 20g of p-nitrophenol and 38g of anhydrous sodium carbonate were suspended in 90 ml 1-methyl-2-pyrolidone under nitrogen atmosphere. 17.3 ml of propargyl bromide were added by means of a dropping funnel. The reaction mixture was heated to 100° C for 60 min. Then the mixture was pored into water and extracted four times with ethyl acetate. The combined organic phases were washed with 1n NaOH and with NaCl solution, dried with Na₂SO₄ followed by concentration at a rotary evaporator to form 23.6g of raw product. The product was recrystallized in isopropanol. Yield 21.8g (85% of the theory) of yellowish crystals, m.p. 113-116°C.

### b) 4-propargyloxy-phenylamin

A 1 liter four necked reaction flask equipped with a thermometer a reflux condensor and a magnetic stirrer was charged with 21g (118 mmol) of 4-nitrophenyl-propargylic-ether (Example 2a) in 295 ml of methanol. 295 ml of concentrated hydrochloric acid was slowly added with rigorous stirring, followed by portions of totally 59g of tin powder. After this addition the reaction temperature was kept at 60° C for 2 hours. The mixture was pored into a solution of 295g of sodium hydroxide in 1.2 l of water. Methanol was distilled off at a rotary evaporator. The suspension was filtered and washed with ethanol. The filtrate was concentrated and distilled in the Kugelrohr apparatus at 180° C /0.07 mbar to yield 10.2g of product. This was recrystallized in a little ethanol to yield 6.8g of white crystals of pure product. MS: 147(M+), 108 (100%), 80, 53.

### c) 5-{(4-propargyloxy-phenylamine)-methylene}-1,3-diisopropyl-pyrimidine-2,4,6-trione.

A 50 ml three necked reaction flask equipped with a thermometer a reflux condensor and a magnetic stirrer under nitrogen atmosphere was charged with 1.45g (10 mmol) of 4-propargyloxy-phenylamin (Example 2b), 2.1g (10 mmol) of 1,3-diisopropyl-pyrimidine-2,4,6-trione (Example 1c) and 2.5 ml of triethyl orthoformate in 15 ml of tetrahydrofuran. This mixture was refluxed for 3.5 hours followed concentrating at a rotary evaporator. The raw product was recrystallized in toluene and hexane to yield 2.9 g of yellow crystals. M.p. 110 - 114°C, UV 352 nm (31'596). This product was irradiated in high dilution with a Hg-lamp 150 W from Hereus and has been shown to be photostable.

### Example 3

### 5-{[4-(2-(1,3,3,3-tetramethyl-1-[(trimethylsilyl)-oxy]-disiloxanyl)-prop-2-enyloxy)-phenylamine]-methylene}-1,3-diisopropyl-pyrimidine-2,4,6-trione.

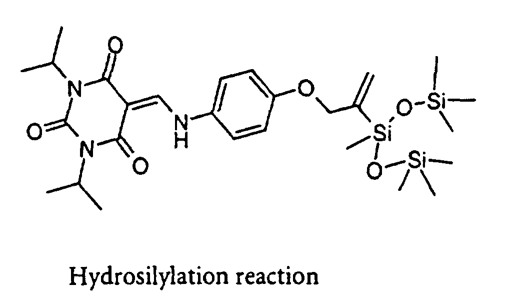

550 mg of the above 5-{(4-propargyloxy-phenylamine)-methylene}-1,3-diisopropyl-pyrimidine-2,4,6-trione (Example 2c), 330 mg of 1,1,1,3,5,5,5-heptamethyl trisiloxane and a catalytic amount of divinyl-tetramethyl disiloxane platinum complex in 10 ml of toluene was placed in a three-necked reaction flask under inert atmosphere and stirred for 20 hours at 80°C. The product solution was washed with a mixture of water/methanol = 1:10 and concentrated to yield 920 mg. This product was chromatographed in hexane ethylacetate = 7:1 to yield 650 mg (76% of the theory) of a honey like liquid. UV 354 nm (ε=31'520), MS: 591 (M⁺), 576, 551, 331 (100%) and 221. Its NMR shows a mixture of the vicinal and the geminal hydrosilylation product = 1.3. It has an unlimited solubility in the cosmetic solvents tested above and excellent photostability qualities in high dilution, determined as described in example 1.

### Example 4

A polysiloxane which corresponds in its statistical mean to the following formula:

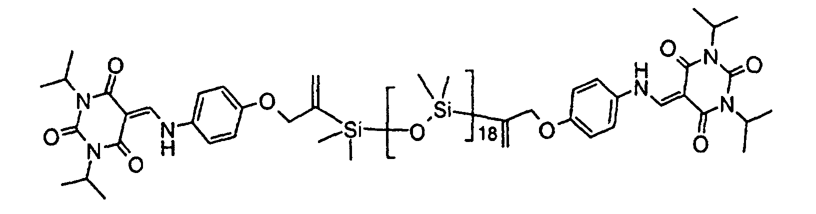

370 mg of the above 5- {(4-propargyloxy-phenylamine)-methylene}-1,3-diisopropyl-pyrimidine-2,4,6-trione (Example 2c), 810 mg of polysiloxane SLM/4 of Wacker-Chemie GmbH and a catalytic amount of divinyl-tetramethyl disiloxane platinum complex in 10 ml of toluene was placed in a three-necked reaction flask under inert atmosphere and heated for 11 days to 80° C. The product solution was washed with a mixture of water/methanol = 1:10 and concentrated to yield 1170 mg (99%) of a dark liquid. UV 355 nm (E=237), having unlimited solubility in Cétiol LC and Crodamol DA and excellent photostability qualities in high dilution, determined as described in Example 1.

### Example 5

A polysiloxane which corresponds in its statistical mean to the following formula:

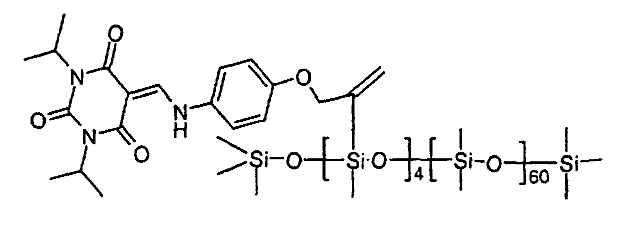

370 mg of the above 5-{(4-propargyloxy-phenylamine)-methylene}-1,3-diisopropyl-pyrimidine-2,4,6-trione (Example 2c), 1100 mg of polysiloxane Ae-151 of Wacker-Chemie GmbH and a catalytic amount of platinum carbon 5% in 10 ml of xylene was placed in a three-necked reaction flask under inert atmosphere and heated for five days to 120°C. The product solution was filtered through Cellite, washed with a mixture of water /methanol = 1:10 and concentrated to yield 1.4 g (95%) of a brownish oil. UV 355 nm (E=189), having unlimited solubility in Cétiol LC and Crodamol DA and excellent photostability qualities in high dilution, determined as described in Example 1.

### Example 6

### 5-{(4-hydroxy-phenylamine)-methylene}-1,3-diisopropyl-pyrimidine-2,4,6-trione

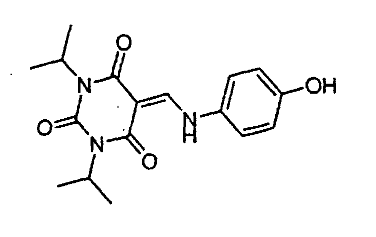

A 150 ml three necked reaction flask equipped with a thermometer a reflux condensor and a magnetic stirrer was charged under nitrogen atmosphere with 3.3 g (30 mmol) of 4-aminophenol, 6.4 g (30 mmol) of 1,3-diisopropyl-pyrimidine-2,4,6-trione (Example 1c) and 7.11 g (50 mmol) of triethyl orthoformate in 60 ml of tert. butylmethyl ether. This mixture was refluxed for 5 hours followed by washing twice with water, dried with Na₂SO₄ and concentrated at a rotary evaporator. The yellow raw product was recrystallized from toluene /hexane to yield 9.5 g of yellow crystals (96% of the theory). M.p.172 - 173°C, UV 351 nm (27'911). This product was irradiated in high dilution with a Hg-lamp 150 W from Hereus and has been shown to be photostable. The solubility in Crodamol DA was found to be 3.6%.

### Example 7

### 5-{(2,4-dihydroxy-phenylamine)-methylene}-1,3-diisopropyl-pyrimidine-2,4,6-trione.

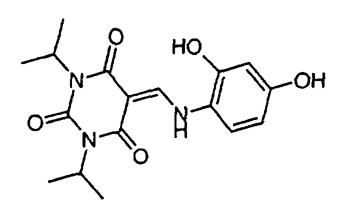

A 50 ml three necked reaction flask equipped with a thermometer a reflux condensor and a magnetic stirrer was charged under nitrogen atmosphere with 3.0 g (19 mmol) of 2,4-dihydroxyaniline, 3.9 g (19 mmol) of 1,3-diisopropyl-pyrimidine-2,4,6-trione (Example 1c) and 5.7 g (39 mmol) of triethyl orthoformate in 15 ml of tert. butyl-methyl ether. This mixture was refluxed for 5.5 hours followed by washing twice with water, dried with Na₂SO₄ and concentrated at a rotary evaporator. The yellow raw product was recrystallized from toluene hexane to yield 2.4 g of brown crystals. M.p.225 - 226°C, UV 363 nm (23'564). This product was irradiated in high dilution with a Hg-lamp 150 W from Hereus and has been shown to be photostable. The solubility in Crodamol DA was found to be 4.1 %.

### Example 8

### 5-{(4-capryloxy-phenylamine)-methylene}-1,3-diisopropyl-pyrimidine-2,4,6-trione.

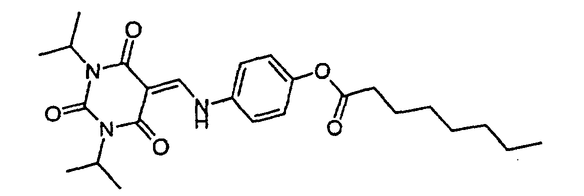

A 50 ml three necked reaction flask equipped with a thermometer a reflux condensor and a magnetic stirrer was charged under nitrogen atmosphere with 1.66 g (5 mmol) of 5-{(4-hydroxy-phenylamine)-methylene}-1,3-diisopropyl-pyrimidine-2,4,6-trione (see Example 6) and 0.48 g (6 mmol) of pyridine in 25 ml of dichloromethane. Then 0.9 g (5.5 mmol) of caprylic acid chloride was slowly added. The reaction started exothermically. The reaction mixture was later heated to 75° C for 22 hours. The mixture was washed twice with each of 1n HCl, NaCl solution and water and backwashed with dichloromethane, dried with Na₂SO₄ and concentrated at a rotary evaporator. The yellow slowly crystallizing oil was recrystallized from hexane to yield 3.2 g of yellowish crystals (70% of the theory). M.p.74 - 76°C, UV 346 nm (33'768). This product was also irradiated in high dilution with a Hg-lamp 150 W from Hereus and has been shown to be photostable. The solubility in Crodamol DA was found to be 18.5 % and in Cétiol LC 6.6%.

### Example 9

### 5-{[(2,4-Bis-capryloxy)-phenylamine])-methylene}-1,3-diisopropyl-pyrimidine-2,4,6-trione and 5-{[(2-hydroxy-4-capryloxy)-phenylamine])-methylene}-1,3-diisopropyl-pyrimidine-2,4,6-trione.

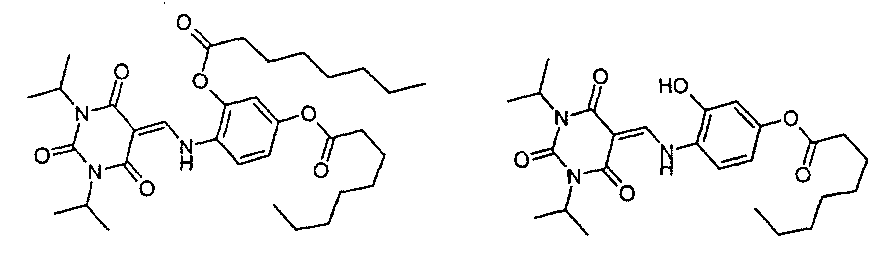

The reaction of Example 8 was repeated using the product of Example 7 (5-{(2,4-dihydroxy-phenylamine)-methylene}-1,3-diisopropyl-pyrimidine-2,4,6-trione). The resulting mixture was chromatographed in hexane /methyl-tert.butyl ether to yield two products, the diester in 85% yield, which was a yellowish honey, UV 351 nm (31'349) and easily mixable with Cétiiol LC in any proportion and the crystalline 5-{[(2-hydroxy-4-capryloxy)-phenylamine])-methylene}-1,3-diisopropyl-pyrimidine-2,4,6-trione in 15% yield, m.p. 115 117°C, UV 354 nm (25'813).

### Example 10

Broad spectrum sunscreen lotion containing 2% of a compound of Example 1.

| wt% | Compound | INCI name | Supplier |
|---|---|---|---|
| | | | |

| **Part A** | | | |
|---|---|---|---|
| 2.00 | PARSOL MCX | Octyl methoxycinnamate | 1 |
| 2.00 | Product of Example 1 | | |
| 3.00 | PARSOL 1789 | 4-tert. Butyl-4-methoxy-dibenzoyl-methane | 1 |
| 12.00 | CETIOL LC | Coco-caprylate/caprate | 8 |
| 4.00 | DERMOL185 | Isostearyl neopentanoate | 9 |
| 0.25 | Diethyleneglycolmonostearate | PEG-2-stearate | 4 |
| 1.00 | Cetylalcohol | Cetylalcohol | |
| 0.25 | MPOB/PPOB | Methyl-propylparabene | |
| 0.10 | EDTA BD | EDTA-sodium salt | 5 |
| 1.00 | AMPHISOL DEA | Diethanolamine cetylphosphate | 1 |

| **Part B** | | | |
|---|---|---|---|
| 20.00 | PERMULENE TR-1 (+%) | Acrylate C10-C30 Alkylacrylate | 7 |
| 48.60 | Deionized Water | Deionized Water | |
| 5.00 | Propyleneglycol | 1,2-Propanediol | |
| 0.80 | KOH (10%) | Potassium hydroxide | |
| INCI: International Nomenclature Cosmetic ingredients | | | |

Part A was heated in a reactor to 85° C. Part B was slowly added within 10 min., followed by addition of KOH, cooling and degassing of the emulsion.

### Example 11

Broad spectrum sunscreen lotion containing 4% of a compound of Example 1.

| wt% | Compound | INCI name | Supplier |
|---|---|---|---|
| | | | |

| **Part A** | | | |
|---|---|---|---|
| 3.00 | PARSOL MCX | Octyl methoxycinnamate | 1 |
| 4.00 | Product of Example 1 | | |
| 3.00 | PARSOL 500 | 4-Methylbenzylidene camphor | 1 |
| 4.00 | PARSOL 1789 | 4-tert. Butyl-4-methoxy-dibenzoyl-methane | 1 |
| 2.00 | Glyceryl monostearate | Glyceryl stearate | |
| 2.00 | Cetyl alcohol extra | Cetyl alcohol | |
| 2.00 | GANEX V-220 | PVP/Eicosene copolymer | 10 |
| 4.00 | CERAPHYL 375 | Isostearyl neopentanoate | 10 |
| 4.00 | CERAPHYL 847 | Octyldodecyl stearoyl stearate | 10 |
| 2.00 | AMPHISOL K | Potassium cetylphosphate | 1 |
| 0.10 | EDETA BD | Disodium EDTA | 5 |
| 0.60 | PHENONIP | Phenoxyethanol & Methyl-, Ethyl-, Propyl- & Butyl-paraben | 6 |

| **Part B** | | | |
|---|---|---|---|
| 11.15 | Deionized Water | Deionized Water | |
| 50.00 | CARBOPOL 934 1% solution | Carbomer | 7 |
| 5.00 | Propyleneglycol | 1,2-Propanediol | |
| 0.15 | NIPAGIN M | Methylparaben | 6 |
| 3.00 | KOH (10%) | Potassium hydroxide | |
| q.s. | Perfume oil | Fragrance | |

Part A was heated in a reactor to 85°C. When homogeneous Part B was added followed by addition of preheated KOH (75°C), cooling and degassing of the emulsion.

### Example 12

Broad spectrum sunscreen cream containing 4% of the product described in Example 5. The cream has a low skin penetration quality:

| wt% | Compound | INCI name | Supplier |
|---|---|---|---|
| | | | |

| **Part A** | | | |
|---|---|---|---|
| 8.00 | PARSOL SLX | Dimethico-diethylbenzalmalonate | 1 |
| 4.00 | Product of Ex. 5 | | |
| 6.00 | T-COTE 031 | Titanium Dioxide & Dimethicone | 3 |
| 10.00 | ESTOL GTEH 3609 | Trioctanoin | 4 |
| 1.00 | Cetyl Alcohol | Cetyl Alcohol | |
| 4.00 | ESTOL GMM 3650 | Glyceryl Myristate | 4 |
| 0.05 | Butylated Hydroxytoluene | BHT | |
| 0.10 | EDETA BD | Disodium EDTA | 5 |
| 0.60 | PHENONIP | Phenoxyethanol & Methyl-, Ethyl-Propyl- & Butyl-paraben | 6 |
| 2.00 | AMPHISOL K | Potassium Cetyl Phosphate | 1 |

| **Part B** | | | |
|---|---|---|---|
| 50.75 | Deionized Water | Deionized Water | |
| 10.00 | CARBOPOL 980 1 % sol'n | Carbomer 980 | 7 |
| 3.00 | Glycerin | Glycerin | |

| **Part C** | | | |
|---|---|---|---|
| 0.50 | KOH 10 % sol'n | Potassium Hydroxide | |
| q.s | Perfume Oil | Fragrance | 2 |

Part A was heated to 85°C while stirring and then mixed for 30 sec. with a turbine at 8000 t/min. When homogeneous, Parts B and C were pre-heated to 75°C and added to Part A. The mixture was cooled to 40° C and Part D was added. The water loss was compensated and stirring was continued while cooling to ambient temperature. Then mixed 30 sec. with a turbine at 8000 t/min

### Suppliers

1) F. HOFFMANN - LA ROCHE LTD, CH-4070 Basel / Switzerland
2) GIVAUDAN - ROURE SA, F-95101 Argenteuil - Paris /France
3) SUNSMART, Wainscott - NY 11975/USA
4) UNICHEMA CHEMIE GmbH, D-4240 Emmerich / Germany
5) BASF AG, D-6700 Ludwigshafen /Germany
6) NIPA LABORATORIES LTD, Mid Glam. - CF38 2SN / England
7) B.F. GOODRICH COMPANY, Brecksville - OH 44141 / USA
8) HENKEL K.G, Düsseldorf/Germany
9) BERNEL Chemical Co. INC. Englwood NJ. USA
10) International Specialty Products ISP

## Claims

1. A compound of the general formula I 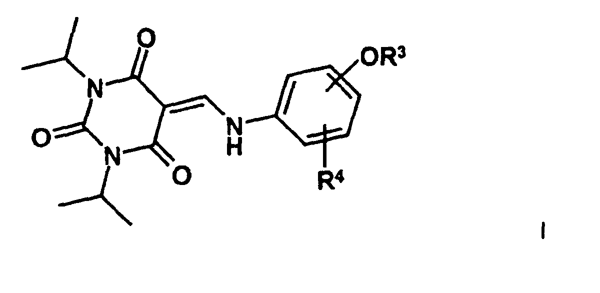 wherein
R³ is hydrogen, C₁-C₁₈ alkyl or C₂-C₁₈ alkyl in which at least one methylene group is replaced by oxygen, or is C₁-C₁₈ alkyl carbonyl, C₃-C₁₈ alkenyl, C₃-C₁₈ alkynyl or a group YS;
R⁴ is hydrogen, C₁-C₈ alkyl or a group OR³;
Y is a linker group which is a C₃-C₁₂ divalent alkylene or alkenylene chain each of which may optionally be interrupted by one or several oxygen atoms; and
S is a silane-, an oligosiloxane- or a polysiloxane moiety, wherein
the silane moiety is a group -SiR⁵R⁶R⁷, wherein R⁵, R⁶ and R⁷ each independently are C₁-C₆ alkyl or phenyl,
the oligosiloxane moiety is a group of the general formula -SiR⁸ₘ(OSiR^{8'}₃)ₙ with m = 0, 1 or 2; n = 1, 2 or 3 and m + n = 3, wherein R⁸ and R^{8'} each independently are C₁-C₆ alkyl or phenyl, or a group of the general formulae IIa or IIb 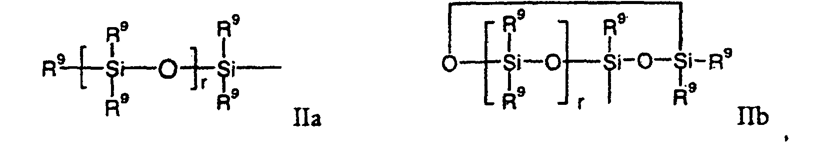
wherein R⁹ is C₁-C₆ alkyl or phenyl and r is an integer from 1 to 9,
and the polysiloxane moiety is a group of the general formulae IIIa or IIIb, 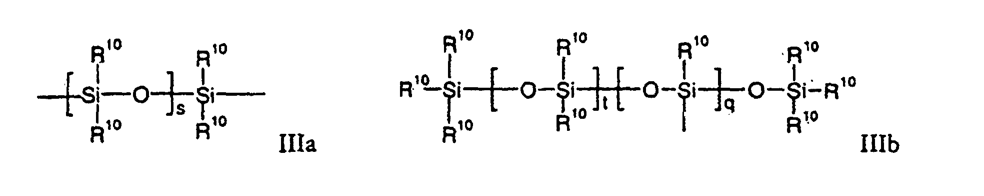 wherein
R¹⁰ is C₁-C₆ alkyl or phenyl;
s is an integer from 4 to 250;
t is an integer from 5 to 250;
q is an integer from 1 to 30.

2. A compound according to claim 1, wherein R⁴ is hydrogen or hydroxy.

3. A compound according to claim 1 or 2, wherein OR³ is in the para position and R³ is 2-ethyl hexyl.

4. A compound according to claim 1 or 2, wherein OR³ is in the para position and R³ is YS.

5. A compound according to claim 4, wherein Y is 3-propylene, 4-butylene, 2-propen-2-ylene, 2-propen-3-ylene or 3-buten-4-ylene.

6. A compound according to claim 4, wherein S is a group -SiR⁵R⁶R⁷, wherein R⁵, R⁶ and R⁷ each independently are C₁-C₆ alkyl or phenyl.

7. A compound according to claim 6, wherein S is trimethylsilane, triethylsilane, tripropylsilane, triisopropylsilane, dimethyl tert.butylsilane, dimethyl thexylsilane, triphenylsilane or dimethylphenylsilane.

8. A compound according to claim 4, wherein S is a group of the general formula -SiR⁸ₘ(OSiR^{8'}₃)ₙ with m = 0, 1 or 2; n = 1, 2 or 3 and m + n = 3, wherein R⁸ and R^{8'} each independently are C₁-C₆ alkyl or phenyl.

9. A compound according to claim 8, wherein S is -SiCH₃(OSi(CH₃)₃)₂.

10. A compound according to claim 4, wherein S is a group of the general formulae IIa or IIb 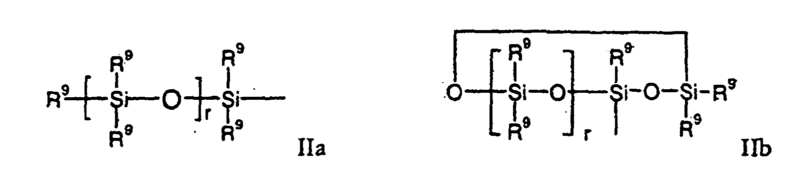 wherein R⁹ is C₁-C₆ alkyl or phenyl and r is an integer from 1 to 9.

11. A compound according to claim 10, wherein R⁹ is methyl.

12. A compound according to claim 4, wherein S is a group of the general formulae IIIa or IIIb, 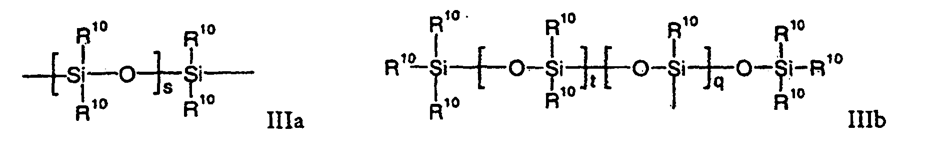 wherein
R¹⁰ is C₁-C₆ alkyl or phenyl;
s is an integer from 4 to 250;
t is an integer from 5 to 250;
q is an integer from 1 to 30.

13. A compound according to claim 12, wherein
R¹⁰ is C₁-C₄ alkyl, preferably methyl;
s is an integer from 4 to 150;
t has a statistical mean value of about 60;
q has a statistical mean value of about 4.

14. A light screening composition comprising a compound according to any of claims 1-13.

15. A light screening composition according to claim 14, further comprising a conventional UV-A and/or UV-B screening agent.

16. The use of a compound according to any of claims 1-13 as absorber for ultraviolet light.

## Patentansprüche

1. Verbindung der allgemeinen Formel 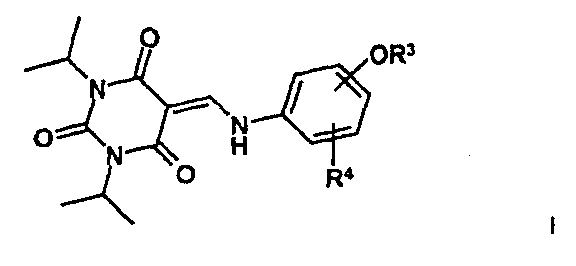 worin
R³ Wasserstoff, C₁-C₁₈-Alkyl oder C₂-C₁₈-Alkyl ist, wobei mindestens eine Methylengruppe durch Sauerstoff ersetzt ist, oder C₁-C₁₈-Alkylcarbonyl, C₃-C₁₈-Alkenyl, C₃-C₁₈-Alkinyl oder eine Gruppe YS ist;
R⁴ Wasserstoff, C₁-C₈-Alkyl oder eine Gruppe OR³ ist;
Y eine Verbindungsgruppe ist, die eine zweiwertige C₃-C₁₂-Alkylen- oder -Alkenylenkette ist, die jeweils gegebenenfalls durch eine oder mehrere Sauerstoffatome unterbrochen sein kann; und
S eine Silan-, eine Oligosiloxan- oder eine Polysiloxankomponente ist, wobei die Silankomponente eine Gruppe -SiR⁵R⁶R⁷ ist, worin R⁵, R⁶ und R⁷ jeweils unabhängig C₁-C₆-Alkyl oder Phenyl sind,
die Oligosiloxankomponente eine Gruppe der allgemeinen Formel -SiR⁸ₘ(OSiR^{8'}₃)ₙ, wobei m = 0, 1 oder 2; n = 1, 2 oder 3 und m + n = 3, wobei R⁸ und R^{8'} jeweils unabhängig C₁-C₆-Alkyl oder Phenyl sind, oder eine Gruppe der allgemeinen Formel IIa oder IIb ist 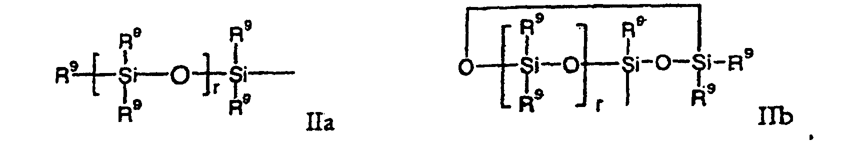
worin R⁹ C₁-C₆-Alkyl oder Phenyl ist und r eine ganze Zahl von 1 bis 9 ist,
und die Polysiloxankomponente eine Gruppe der allgemeinen Formeln IIIa oder IIIb ist 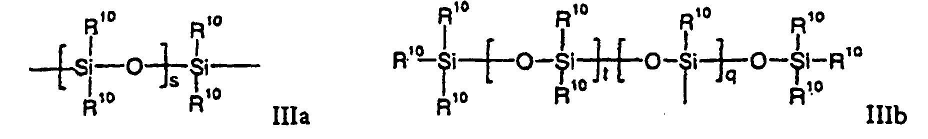 worin
R¹⁰ C₁-C₆-Alkyl oder Phenyl ist,
s eine ganze Zahl von 4 bis 250 ist,
t eine ganze Zahl von 5 bis 250 ist,
q eine ganze Zahl von 1 bis 30 ist.

2. Verbindung nach Anspruch 1, wobei R⁴ Wasserstoff oder Hydroxy ist.

3. Verbindung nach Anspruch 1 oder 2, wobei OR³ in der para-Stellung ist und R³ 2-Ethylhexyl ist.

4. Verbindung nach Anspruch 1 oder 2, wobei OR³ in der para-Stellung ist und R³ YS ist.

5. Verbindung nach Anspruch 4, wobei Y 3-Propylen, 4-Butylen, 2-Propen-2-ylen, 2-Propen-3-ylen oder 3-Buten-4-ylen ist.

6. Verbindung nach Anspruch 4, wobei S eine Gruppe -SiR⁵R⁶R⁷ ist, worin R⁵, R⁶ und R⁷ jeweils unabhängig C₁-C₆-Alkyl oder Phenyl sind.

7. Verbindung nach Anspruch 6, wobei S Trimethylsilan, Triethylsilan, Tripropylsilan, Triisopropylsilan, Dimethyltertbutylsilan, Dimethylthexylsilan, Triphenylsilan oder Dimethylphenylsilan ist.

8. Verbindung nach Anspruch 4, wobei S eine Gruppe der allgemeinen Formel -SiR⁸ₘ(OSiR^{8'}₃)ₙ ist, wobei m = 0, 1 oder 2; n = 1, 2, oder 3 und m + n = 3, wobei R⁸ und R^{8'} jeweils unabhängig C₁-C₆-Alkyl oder Phenyl sind.

9. Verbindung nach Anspruch 8, wobei S -SiCH₃(OSi(CH₃)₃)₂ ist.

10. Verbindung nach Anspruch 4, wobei S eine Gruppe der allgemeinen Formeln IIa oder IIb ist 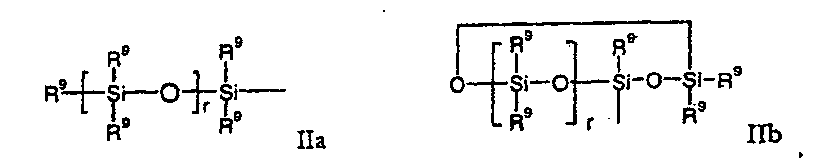 worin R⁹ C₁-C₆-Alkyl oder Phenyl ist und r eine ganze Zahl von 1 bis 9 ist.

11. Verbindung nach Anspruch 10, wobei R⁹ Methyl ist.

12. Verbindung nach Anspruch 4, wobei S eine Gruppe der allgemeinen Formeln IIIa oder IIIb ist 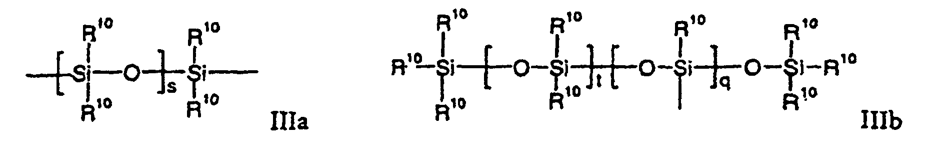 worin
R¹⁰ C₁-C₆-Alkyl oder Phenyl ist,
s eine ganze Zahl von 4 bis 250 ist,
t eine ganze Zahl von 5 bis 250 ist,
q eine ganze Zahl von 1 bis 30 ist.

13. Verbindung nach Anspruch 12, wobei
R¹⁰ C₁-C₄-Alkyl, vorzugsweise Methyl ist,
s eine ganze Zahl von 4 bis 150 ist,
t einen statistischen Mittelwert von etwa 60 aufweist,
q einen statistischen Mittelwert von etwa 4 aufweist.

14. Lichtschutzzusammensetzung, umfassend eine Verbindung nach einem der Ansprüche 1 bis 13.

15. Lichtschutzzusammensetzung nach Anspruch 14, außerdem umfassend ein konventionelles UV-A- und/oder UV-B-Lichtschutzmittel.

16. Verwendung einer Verbindung nach einem der Ansprüche 1 bis 13 als Absorber für UV-Licht.

## Revendications

1. Composé de formule générale I : 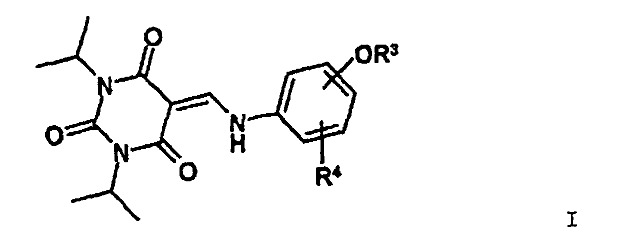 dans laquelle
R³ est un atome d'hydrogène, un groupe alkyle en C₁-C₁₈ ou un groupe alkyle en C₂-C₁₈ dans lequel au moins un groupe méthylène est remplacé par un atome d'oxygène, ou est un groupe alkyle (en C₁-C₁₈)carbonyle, un groupe alcényle en C₃-C₁₈, un groupe alcynyle en C₃-C₁₈ ou un groupe YS;
R⁴ est un atome d'hydrogène, un groupe alkyle en C₁-C₈ ou un groupe OR³;
Y est un groupe de liaison qui est une chaîne alkylène ou alcénylène en C₃-C₁₂ divalente, qui peut être éventuellement interrompue par un ou plusieurs atomes d'oxygène; et
S est un groupe caractéristique silane, oligosiloxane ou polysiloxane, dans lequel le groupe caractéristique silane est un groupe -SiR⁵R⁶R⁷, où R⁵, R⁶ et R⁷ sont chacun indépendamment un groupe alkyle en C₁-C₆ ou un groupe phényle,
le groupe caractéristique oligosiloxane est un groupe de formule générale -SiR⁸ₘ(OSiR^{8'}₃)ₙ avec m = 0, 1 ou 2; n = 1, 2 ou 3 et m + n = 3, dans laquelle R⁸ et R^{8'} sont chacun indépendamment un groupe alkyle en C₁-C₆ on un groupe phényle, ou un groupe de formule générale IIa ou IIb 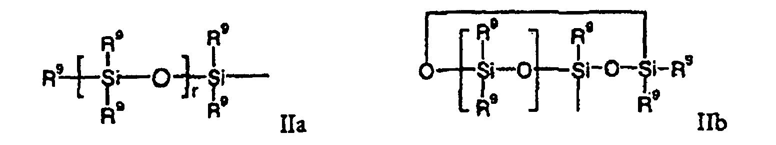 dans laquelle R⁹ est un groupe alkyle en C₁-C₆ ou un groupe phényle, et r est un nombre entier de 1 à 9,
et le fragment polysiloxane est un groupe de formule générale IIIa ou IIIb 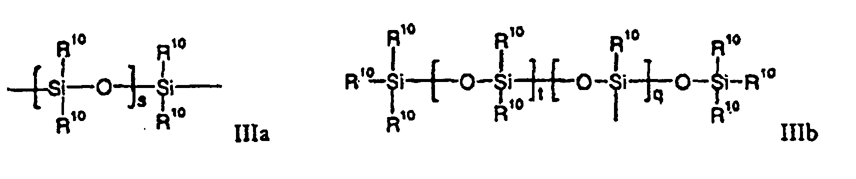 dans laquelle
R¹⁰ est un groupe alkyle en C₁-C₆ ou un groupe phényle ;
s est un nombre entier de 4 à 250 ;
t est un nombre entier de 5 à 250 ;
q est un nombre entier de 1 à 30.

2. Composé selon la revendication 1, dans lequel R⁴ est un atome d'hydrogène ou un groupe hydroxy.

3. Composé selon la revendication 1 ou 2, dans lequel OR³ est en position para et R³ est un groupe 2-éthylhexyle.

4. Composé selon la revendication 1 ou 2, dans lequel OR³ est en position para et R³ est un groupe YS.

5. Composé selon la revendication 4, dans lequel Y est un groupe 3-propylène, 4-butylène, 2-propén-2-ylène, 2-propén-3-ylène ou 3-butèn-4-ylène.

6. Composé selon la revendication 4, dans lequel S est un groupe -SiR⁵R⁶R⁷, où R⁵, R⁶ et R⁷ sont chacun indépendamment un groupe alkyle en C₁-C₆ ou un groupe phényle.

7. Composé selon la revendication 6, dans lequel S est un groupe triméthylsilane, triéthylsilane, tripropylsilane, triisopropylsilane, diméthyl-tert-butylsilane, diméthyl-thexylsilane, triphénylsilane ou diméthylphénylsilane.

8. Composé selon la revendication 4, dans lequel S est un groupe de formule générale -SiR⁸ₘ(OSiR^{8'}₃)ₙ avec m = 0, 1 ou 2 ; n = 1, 2 ou 3 et m + n = 3, dans laquelle R⁸ et R^{8'} sont chacun indépendamment un groupe alkyle en C₁-C₆ ou un groupe phényle.

9. Composé selon la revendication 8, dans lequel S est -SiCH₃(OSi(CH₃)₃)₂.

10. Composé selon la revendication 4, dans lequel S est un groupe de formule générale IIa ou IIb 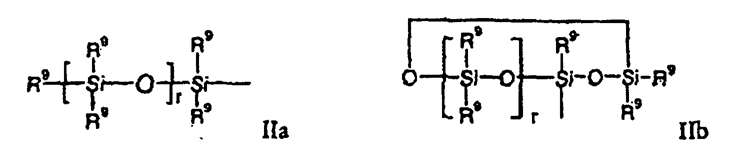 dans laquelle R⁹ est un groupe alkyle en C₁-C₆ ou un groupe phényle, et r est un nombre entier de 1 à 9.

11. Composé selon la revendication 10, dans lequel R⁹ est un groupe méthyle.

12. Composé selon la revendication 4, dans lequel S est un groupe de formule générale IIIa ou IIIb 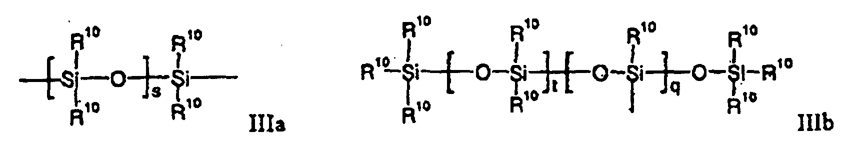 dans laquelle
R¹⁰ est un groupe alkyle en C₁-C₆ ou un groupe phényle ;
s est un nombre entier de 4 à 250 ;
t est un nombre entier de 5 à 250 ;
q est un nombre entier de 1 à 30.

13. Composé selon la revendication 12, dans lequel
R¹⁰ est un groupe alkyle en C₁-C₄, de préférence le groupe méthyle ;
s est un nombre entier de 4 à 150 ;
t a une valeur moyenne statistique d'environ 60 ;
q a une valeur moyenne statistique d'environ 4.

14. Composition de protection contre la lumière comprenant un composé selon l'une quelconque des revendications 1 à 13.

15. Composition de protection contre la lumière selon la revendication 14, comprenant en outre un agent de protection anti-UV-A et/ou anti-UV-B conventionnel.

16. Utilisation d'un composé selon l'une quelconque des revendications 1 à 13 comme absorbant de rayons ultraviolets.
